# EUROPÄISCHE PATENTSCHRIFT

(11) **EP 1 245 193 B1**
(45) Veröffentlichungstag und Bekanntmachung des Hinweises auf die Patenterteilung: **03.11.2004**
(21) Anmeldenummer: 01810324.2
(22) Anmeldetag: 29.03.2001
(51) Int. Cl.: A61B 17/02

(54) **Spreizvorrichtung für Kniegelenke**
Knee distraction device
Appareil d'écartement pour les articulations de genou

(43) Veröffentlichungstag der Anmeldung: 02.10.2002
(73) Patentinhaber: Centerpulse Orthopedics Ltd., 6340 Baar (CH)
(72) Erfinder: Overes, Tom, 8400 Winterthur (CH); Gyssler, Bernhard, 8810 Horgen (CH)
(74) Vertreter: Manitz, Finsterwald & Partner Gbr

(56) Entgegenhaltungen:
- EP-A- 0 809 969
- US-A- 3 200 501
- US-A- 4 501 266
- US-A- 5 669 914

## Beschreibung

Die Erfindung handelt von einer Spreizvorrichtung für Kniegelenke mit zwei parallelen Stützscheiben, die zwischen Femurkondylen und Tibia einsteckbar sind und die mittels eines Verstellmechanismus, welcher ein Gehäuse aufweist, auseinander fahrbar sind, gemäß dem Oberbegriff des Anspruchs 1.

Spreizvorrichtungen an Gelenken dienen dem Aufspreizen eines freigelegten Gelenks, um an diesem bestimmte chirurgische Arbeiten vorzunehmen. Bei Kniegelenken haben solche Vorrichtungen zwei parallele Stützscheiben die zwischen Tibia- und Femurkondylen eingeschoben werden und dann mit Hilfe eines Verstellmechanismus auseinander gefahren werden.

Eine Spreizvorrichtung dieser Art wird von der Firma Sulzer Orthopedics Inc., 9900 Spectrum Drive, Austin, TX 7871, USA, hergestellt. Die Stützscheiben werden mittels einer Zahnstange, in deren Zahnung einer Federklinke zur Sicherung der erreichten Spreizhöhe einrastet, auseinander bewegt. Die Höhenverstellung der Zahnstange erfolgt über ein Zahnrad, welches in die Zahnstange eingreift und über eine Kurbel angetrieben werden kann. Ein Nachteil dieser Einrichtung besteht darin, dass der Operateur über den Zustand an den Bändern keine Informationen hat. In der US-A-5,669,914 wird eine Spreizvorrichtung für Kniegelenke gezeigt, die zwischen dem Verstellmechanismus und Stützscheiben elastisch federnde Übertragungsglieder enthält, um gleichzeitig Spreizweg und Spreizkraft festzustellen. Ein Nachteil dieser Einrichtung besteht darin, dass wegen des Eingriffs wesentliche Bänder bei diesen Messungen nicht beteiligt sind.

Aufgabe der Erfindung ist es, in dieser Richtung mit einer verbesserten Spreizvorrichtung Abhilfe zu schaffen. Diese Aufgabe wird gemäss den Merkmalen des unabhängigen Anspruchs 1 erfüllt.

Der Vorteil der Erfindung besteht darin, dass die Reaktionskräfte der Bänder während dem Spreizen direkt ablesbar sind und mit einer ebenfalls ablesbaren Spreizhöhe vergleichbar sind.

Die abhängigen Ansprüche 2 bis 8 stellen vorteilhafte Weiterbildungen der Erfindung dar.

Die Stützscheiben besitzen eine hintere Aussparung um noch vorhandene Kreuzbänder zu schonen. Dadurch, dass auf der Vorderseite der Stützscheiben eine weitere Aussparung angebracht ist und die Verbindung zum Ver stellmechanismus über einen seitlich weggehenden Steg erfolgt, ist ein Einschieben der Stützscheiben von der Seite ohne Steg, beispielsweise von lateral, möglich, ohne dass die Patella und die Patellabänder aus ihrer natürlichen Lage entfernt werden müssen. Der Steg ist so in einem Bogen zum Verstellmechanismus geführt, dass dieser mit seiner Vorschubachse in der sagittalen Mittelebene der Stützscheiben liegt. Da die Abstützung einer Stützscheibe beispielsweise der oberen Stützscheibe in einem Scharniergelenk mit horizontalen Achse in dieser Mittelebene erfolgt, kann die obere Stützscheibe nach medial oder lateral wegkippen und dem Operateur zwischen Femur und Tibia die aktuelle Varus-/Valgusstellung anzeigen. Der Operateur hat damit die Möglichkeit die Wirkung der Bänder unter natürlichen Bedingungen zu überprüfen. Die Stützscheiben sind über die Stege durch lösbare Steckverbindungen mit dem Verstellmechanismus verbunden. Diese Steckverbindungen sind ein zweites Mal an der mittleren Sagittalebene gespiegelt vorhanden und symmetrisch ausgeführt damit die gleichen Stützscheiben für ein laterales oder mediales Einbringen verwendbar sind. Die lösbare Steckverbindung ermöglicht es auch paarweise verschieden grosse Stützscheiben zu verwenden. Da der äussere Abstand der Stützscheiben für das Einstecken der Stützscheiben begrenzt ist und zu grosse Biegedeformationen der Stützscheiben unerwünscht sind, kann es vorteilhaft sein, wenn die Stützscheiben in bestimmten Bereichen Rippen und Täler aufweisen, die sich gegenseitig durchdringen und der einzelnen Stützscheibe ein grösseres Widerstandsmoment gegen Biegung geben.

Die eigentliche Höhenverstellung der Spreizhöhe erfolgt mit einer Gewindeschraube, die sich über eine elastische Feder am Gehäuse abstützt und einen Stössel, der die obere Stützscheibe trägt, nach oben oder unten bewegt. Das Gewinde hat den Vorteil, dass eine Feinverstellung der Spreizhöhe und eine Selbsthemmung des Verstellmechanismus gegeben sind.

Um möglichst wenig Platz im Operationsbereich wegzunehmen, ist der Verstellmechanismus so konzipiert, dass er nur wenig über die obere Stützscheibe vorsteht und mit einem schmalen rohrförmigen Gehäuse versehen ist, dessen Durchmesser zweimal in den Abmessungen der Stützscheibe von lateral nach medial Platz hat. Das rohrförmige Gehäuse ist durch eine Drehhülse verlängert, die bezüglich Drehung formschlüssig eine Verlängerung der Gewindeschraube umfasst. An der Drehhülse sind Ansetzflächen angebracht, damit nur während der Verstellung zusätzlich Platz durch ein aufsetzbares Werkzeug zum Beispiel durch einen Gabelschlüssel beansprucht wird. Die Verlängerung, die je nach Beanspruchung der Feder zwischen Gewindeschraube und Gehäuse aus dem Gehäuse vorsteht, erlaubt es, die Federkraft direkt an einer Skala der Drehhülse abzulesen.

Ein weiterer Vorteil der Konstruktion mit auswechselbaren Stützscheiben besteht darin, dass auch monokondylare Stützscheiben für nur eine Gelenkhälfte verwendet werden können. Allerdings muss in einem solchen Fall das Scharniergelenk blockiert werden, oder die Verbindungsstege müssen so zum Verstellmechanismus geführt werden, dass die Scharnierachse in die mittlere Sagittalebene der monokondylären Stützscheiben zu liegen kommt.

Eine weitere Anwendung besteht darin, Schnittblöcke auf den Stützscheiben in vorbestimmten Positionen zu befestigen, um in einer als optimal gefundenen Position Resektionsschnitte an Knochenteilen vorzunehmen.

Im folgenden wird die Erfindung anhand von Ausführungsbeispielen beschrieben. Es zeigen:
- Fig. 1: Schematisch einen Längsschnitt durch ein linkes Kniegelenk mit der Seitenansicht einer von lateral eingeführten Spreizvorrichtung;
- Fig. 2: schematisch und vergrössert eine Ansicht schräg von unten auf eine Spreizvorrichtung mit Stützscheiben gemäss Fig. 1;
- Fig. 3: schematisch eine Frontansicht auf eine Spreizvorrichtung gemäss Fig. 2 ohne Stützscheiben;
- Fig. 4: schematisch einen Längsschnitt durch die Spreizvorrichtung von Fig. 3;
- Fig. 5: schematisch eine Draufsicht auf eine Spreizvorrichtung gemäss Fig. 2;
- Fig. 6: schematisch einen Längsschnitt durch die Stützscheiben von Fig. 5; und
- Fig. 7: schematisch einen Querschnitt durch die Stützscheiben von Fig. 5.

Mit den Figuren sind Spreizvorrichtungen für Kniegelenke gezeigt, die zwei parallele Stützscheiben 2, 3 aufweisen, welche zwischen Femurkondylen 4, 5 und Tibia 6 einsteckbar sind und welche mit einem Verstellmechanismus 10 verbunden auseinanderfahrbar sind. Der Verstellmechanismus 10 besitzt ein Gehäuse 1 auf dem sich ein hubverlängernder Stössel 17 über ein elastisch federndes Übertragungsglied 7 abstützt, um gleichzeitig den effektiven Spreizweg an einem Massstab 8 am Gehäuse 1 und die Grösse der Spreizkraft an einem Massstab 9 am Gehäuse 1 ablesbar zu machen.

In den nachfolgenden Figuren sind gleiche Hinweiszeichen für gleiche Funktionen verwendet.

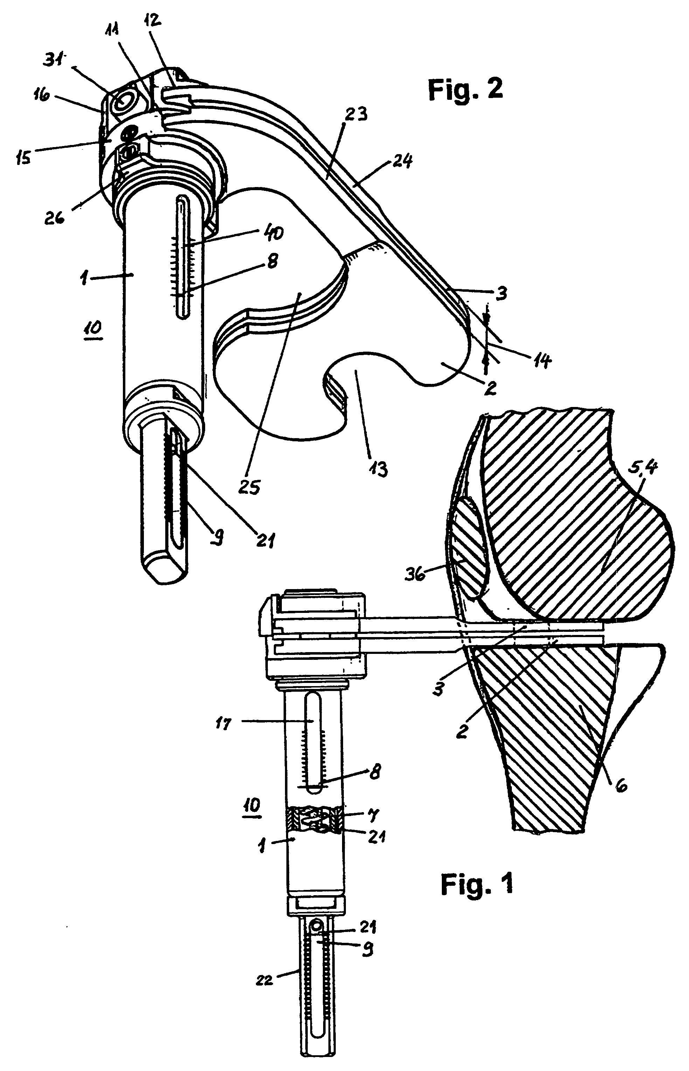

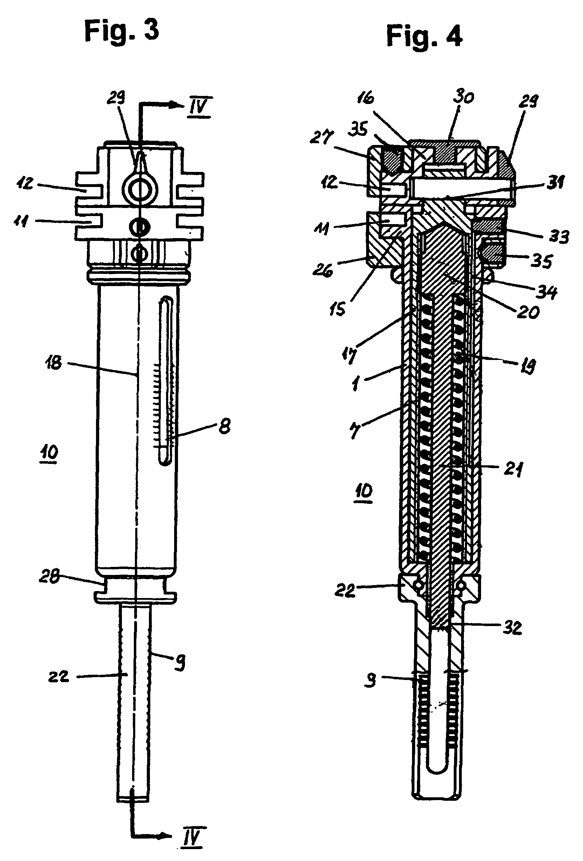

Im Beispiel der Figuren 1 und 2 ist ein Paar von Stützscheiben 2, 3 zwischen den Femurkondylen 4, 5 und der Tibia 6 hinter der Patella 36 eingeschoben und mit einem Verstellmechanismus 10 verbunden, der an seinem Gehäuse 1 einen oberen Massstab 8 aufweist, welcher durch einen Schlitz 40 die Sicht auf einen Stössel 17 und dessen Markierung freigibt, um den effektiven Stösselhub ablesbar zu machen. Auf der Unterseite des rohrförmigen Gehäuses 1 ist eine Drehhülse 22 drehbar am Gehäuse gelagert. Die Drehhülse 22 ist mit einem Schlitz versehen, an dessen Rändern ein Massstab 9 für eine Federkraft angebracht ist. Eine Verstellschraube 20, die in eine Gewindebohrung 19 (Fig. 4) des Stössels 17 eingreift, treibt die beiden Stützscheiben 2, 3 auseinander. Entsprechend dem Widerstand der Gelenkbänder wird ein elastisch federndes Übertragungsglied 7 in Form einer Schraubenfeder, die die Verstellschraube 20 gegen das Gehäuse 1 abstützt, zusammengepresst. Da die Verstellschraube 20 eine Verlängerung 21 aufweist, die bis in die Drehhülse 22 hineinreicht, kann über die Lage dieser Verlängerung 21 die an der Schraubenfeder anliegende Kraft abgelesen werden. Dabei ist diese Verlängerung formschlüssig aber längsverschieblich in der Drehhülse 22 eingepasst, um über eine Drehung an der Drehhülse eine Drehung der Verstellschraube und damit eine Hubverstellung zu bewirken.

In Figur 2 erkennt man die Form der bikondylaren Stützscheiben 2, 3, welche eine vordere Aussparung 25 und eine hintere Aussparung 13 aufweisen. Die laterale Seite der Stützscheiben 2, 3 ist in Form von einem Steg 23, 24 weitergeführt. Die Stützscheiben 2, 3 weisen eine Einsteckhöhe 14 auf und liegen in Nuten am Verstellmechanismus 10 auf, um eine Steckverbindung 11, 12 zu bilden. Die Aufnahmen für die Stege 23, 24 werden für den oberen Steg 24 durch einen Befestigungskopf 16, welcher über ein Scharniergelenk 31 am Stössel 17 verankert ist, und für den unteren Steg 23 durch einen Befestigungskopf 15 gebildet, welcher Bestandteil des Gehäuses 1 ist.

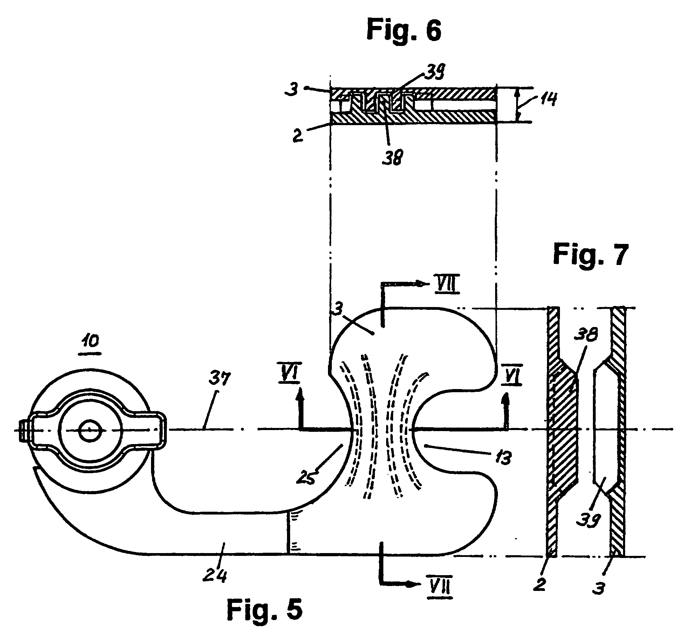

In Figur 3 und 4 ist der Zweck der Komponenten des Verstellmechanismus erkennbar. Der obere Befestigungskopf 16 ist bei ausgefahrenem Stössel 17 um ein Scharniergelenk 31 schwenkbar und kann nach medial oder lateral abkippen. Die Kippbewegung ist relativ zu einem Zeiger 29 ablesbar, der auf der Scharnierwelle befestigt ist. Die eingesteckten Stege 23, 24 der Stützscheiben 2, 3 werden über drehbar6 Sicherungshülsen 26, 27, die jeweils einen in axialer Richtung vorstehenden Schulterabschnitt aufweisen, verriegelt und ihrerseits durch Sicherungselemente 35 zum Beispiel durch Schrauben blockiert. Der in einer Bohrung des Gehäuses 1 längsverschiebliche Stössel 17 ist mit seiner Längsnut 34 durch einen Gewindestift 33 geführt. Die Verstellschraube 20 sitzt in der Gewindebohrung 19 des Stössels 17 und ist darin durch Drehen auf und ab bewegbar. Sie besitzt eine Verlängerung 21, die bis in die Drehhülse 22 hineinragt und dort formschlüssig längsverschiebbar ist. Durch Drehen der Drehhülse beispielsweise an den Ansetzflächen 28 und durch den Formschluss 32 kann der Stössel 17 relativ zur Verstellschraube 20 nach oben verschoben werden, wobei die Stützscheiben auseinanderfahren. Sobald der Stössel 17 mit seiner unteren Kante vom Gehäuse 1 abhebt, liegt er nur noch auf dem federnden Übertragungsglied 7, d.h. auf der Schraubenfeder auf. Bei abgehobenem Stössel 17 ist daher die Schraubenfeder immer entsprechend der Spreizkraft komprimiert, wobei diese Spreizkraft am Massstab 9 der Drehhülse 22 entsprechend der Position des Endes der Verlängerung 21 ablesbar ist. Die Längsachse 18 des Verstellmechanismus 10 liegt in der mittleren Sagittalebene 37 (Fig. 6) der Stützscheiben. Die Schraubenfeder kann für eine Kompressionskraft bis zu 300 Newton ausgelegt sein. In der Praxis sollte ein Bereich von 0 bis 170 Newton ausreichend sein, um die Wirkung der Bänder zu überprüfen. Der obere Befestigungskopf 16 ist durch einen Deckel 30 geschlossen.

Figur 6 zeigt eine besondere Ausführung von Stützscheiben 2, 3, die im Bereich der mittleren Sagittalebene 37 Rippen 38 und Täler 39 aufweisen, die sich gegenseitig durchdringen, um trotz unveränderter Einsteckhöhe 14 ein grösseres Widerstandsmoment zu erzeugen.

## Patentansprüche

1. Spreizvorrichtung für Kniegelenke mit zwei parallelen Stützscheiben (2, 3), die zwischen Femurkondylen (4, 5) und Tibia (6) einsteckbar sind und die mittels eines Verstellmechanismus (10), welcher ein Gehäuse (1) aufweist, auseinanderfahrbar sind, wobei der Verstellmechanismus (10) ein elastisch federndes Übertragungsglied (7) zu einer der Stützscheiben (2, 3) enthält, um gleichzeitig den effektiven Spreizweg an einem Massstab (8) am Gehäuse (1) und die Grösse der Spreizkraft an einem Massstab (9) am Gehäuse (1) ablesbar zu machen, **dadurch gekennzeichnet, dass** die Stützscheiben (1, 2) mit lösbaren Steckverbindungen (11, 12) zum Verstellmechanismus (10) versehen sind und dass die Stützscheiben (2, 3) einen seitlichen Steg zum Verstellmechanismus (10) aufweisen, um ein Aufspreizen unter Mitwirkung von Patella und Patellabändern zu ermöglichen.

2. Spreizvorrichtung nach Anspruch 1, **dadurch gekennzeichnet, dass** die Stützscheiben (2, 3) Aussparungen (13) zu den Kreuzbändern aufweisen.

3. Spreizvorrichtung nach Anspruch 1 oder 2, **dadurch gekennzeichnet, dass** der Verstellmechanismus (10) für eine der Stützscheiben (2, 3) ein Scharniergelenk (31) aufweist, um wie bei einem Wagebalken eine VarusNalgusstellung in der gespreizten Stellung mit einem Zeiger (29) anzuzeigen.

4. Spreizvorrichtung nach einem der Ansprüche 1 bis 3, **dadurch gekennzeichnet, dass** sich die Stützscheiben (2, 3) in der Ausgangsstellung vor dem Spreizen gegenseitig durchdringen, um bei einer vorgegebenen Einsteckhöhe (14) über beide Scheiben (1, 2) ein grösseres Widerstandsmoment bezüglich Biegung für die einzelne Scheibe als das Widerstandsmoment für eine Scheibe mit der halben Einsteckhöhe zu erhalten.

5. Spreizvorrichtung nach einem der Ansprüche 1 bis 4, **dadurch gekennzeichnet, dass** der Verstellmechanismus in beiden Verstellrichtungen "Auseinanderfahren" und "Zusammenfahren" selbsthemmend ist.

6. Spreizvorrichtung nach einem der Ansprüche 1 bis 5, **dadurch gekennzeichnet, dass** der Verstellmechanismus (10) ein rohrförmiges Gehäuse (1) mit einem ersten Befestigungskopf (15) für die erste Stützscheibe (2) und mit einem darin geführten Stössel (17) mit einem zweiten Befestigungskopf (16) für die zweite Stützscheibe (3) aufweist.

7. Spreizvorrichtung nach Anspruch 6, **dadurch gekennzeichnet, dass** der Stössel (17) in der Verschiebeachse (18) mit einer Gewindebohrung (19) versehen ist, in welcher eine Verstellschraube (20) dreht, die ihrer seits über ein federndes Übertragungsglied (7) in Form einer Schraubenfeder am Gehäuse (1) abgestützt ist und die über eine aus dem Gehäuse (1) ragende Verlängerung (21) drehbar ist, wobei die herausragende Verlängerung (21) der Verstellschraube (20) einen Massstab (9) für die Spreizkraft bildet.

8. Spreizvorrichtung nach Anspruch 7, **dadurch gekennzeichnet, dass** die Verlängerung (21) in eine am Gehäuse (1) gelagerte Drehhülse hineinragt, welche bei unterschiedlich weit herausragender Verlängerung (21) die Übertragung eines Drehmoments mittels Formschluss (32) auf die Verlängerung (21) erlaubt.

## Claims

1. A spreader apparatus for knee joints having two parallel support plates (2, 3) which can be inserted between the femur condyles (4, 5) and the tibia (6) and which can be moved apart by means of an adjustment mechanism (10) which has a housing (1), wherein the adjustment mechanism (10) includes an elastically resilient transmission member (7) to one of the support plates (2, 3) in order to simultaneously make the effective spreading travel readable on a scale (8) at the housing (1) and the magnitude of the spreading force readable on a scale (9) at the housing (1), **characterised in that** the support plates (1, 2) are provided with releasable plug connections (11, 12) to the adjustment mechanism (10); and **in that** the support plates (2, 3) have a lateral web to the adjustment mechanism (10) to allow a spreading including the patella and the patella ligaments.

2. A spreader apparatus in accordance with claim 1, **characterised in that** the support plates (2, 3) have recesses (13) with respect to the cruciate ligaments.

3. A spreader apparatus in accordance with claim 1 or claim 2, **characterised in that** the adjustment mechanism (10) has a hinge joint (31) for one of the support plates (2, 3) to indicate a varus/valgus position in the spread position with an indicator (29) as with a scale beam.

4. A spreader apparatus in accordance with any of claims 1 to 3, **characterised in that** the support plates (2, 3) mutually penetrate one another in the starting position prior to spreading in order, with a pre-set insertion height (14), to obtain, over both plates (1, 2), a greater section modulus with respect to bending for the individual plate than the section modulus for a plate with half the insertion height.

5. A spreader apparatus in accordance with any of claims 1 to 4, **characterised in that** the adjustment mechanism is self-locking in both adjustment directions of "moving apart" and "moving together".

6. A spreader apparatus in accordance with any of claims 1 to 5, **characterised in that** the adjustment mechanism (10) has a tubular housing (1) with a first fastening head (15) for the first support plate (2) and with a tappet (17) guided therein with a second fastening head (16) for the second support plate (3).

7. A spreader apparatus in accordance with claim 6, **characterised in that** the tappet (17) (18) is provided in the displacement axis with a threaded bore (19) in which a setting screw (20) rotates which is in turn supported at the housing (1) via a resilient transmission member (7) in the form of a helical spring and which can be rotated via an extension (21) projecting out of the housing (1), with the projecting extension (21) of the setting screw (20) forming a scale (9) for the spreading force.

8. A spreader apparatus in accordance with claim 7, **characterised in that** the extension (21) projects into a rotary sleeve supported at the housing (1) which allows the transmission of a torque to the extension (21) by means of form fitted connection (32) for different projecting amounts of the extension (21).

## Revendications

1. Dispositif écarteur destiné à des articulations de genou, comportant deux disques de soutien (2, 3) parallèles qui peuvent être insérés entre des condyles fémoraux (4, 5) et le tibia (6), et qui peuvent être écartés au moyen d'un mécanisme de réglage (10) comportant un boîtier (1), le mécanisme de réglage (10) contenant un élément de transmission (7) élastiquement souple vers l'un des disques de soutien (2, 3) afin de rendre simultanément lisible la distance d'écartement effective sur une échelle graduée (8) du boîtier (1), et l'ampleur de la force d'écartement sur une échelle graduée (9) du boîtier (1), **caractérisé en ce que** les disques de soutien (1, 2) sont munis de jonctions enfichables amovibles (11, 12) pour le mécanisme de réglage (10), et **en ce que** les disques de soutien (2, 3) comportent une entretoise latérale par rapport au mécanisme de réglage (10) afin de permettre un écartement en association avec la patelle et les ligaments de la patelle.

2. Dispositif écarteur selon la revendication 1, **caractérisé en ce que** les disques de soutien (2, 3) comportent des évidements (13) pour les ligaments croisés.

3. Dispositif écarteur selon la revendication 1 ou 2, **caractérisé en ce que** le mécanisme de réglage (10) comporte une articulation à charnière (31) pour l'un des disques de soutien (2, 3) afin d'indiquer à l'état écarté une position varus / valgus avec un index (29), comme pour un fléau de balance.

4. Dispositif écarteur selon l'une quelconque des revendications 1 à 3, **caractérisé en ce que**, dans la position initiale avant l'écartement, les disques de soutien (2, 3) s'interpénètrent mutuellement afin d'obtenir, par l'intermédiaire des deux disques (1, 2) à une hauteur d'insertion (14) prédéterminée, un couple de résistance plus élevé en ce qui concerne la flexion d'un disque unique que le couple de résistance d'un disque à mi-hauteur d'insertion.

5. Dispositif écarteur selon l'une quelconque des revendications 1 à 4, **caractérisé en ce que** le mécanisme de réglage est autobloquant dans les deux sens de réglage "écartement" et "rapprochement".

6. Dispositif écarteur selon l'une quelconque des revendications 1 à 5, **caractérisé en ce que** le mécanisme de réglage (10) comporte un boîtier tubulaire (1) avec une première tête de fixation (15) pour le premier disque de soutien (2) et, guidé dans celle-ci, un coulisseau (17) avec une deuxième tête de fixation (16) pour le deuxième disque de soutien (3).

7. Dispositif écarteur selon la revendication 6, **caractérisé en ce que** le coulisseau (17) est muni d'un alésage fileté (19) dans l'axe de translation (18), dans lequel tourne une vis de réglage (20) qui prend de son côté appui contre le boîtier (1) par l'intermédiaire d'un élément de transmission élastique (7) sous la forme d'un ressort à boudin, et qui peut être tournée par l'intermédiaire d'un prolongement (21) dépassant du boîtier (1), le prolongement (21) dépassant de la vis de réglage (20) formant une échelle graduée (9) pour la force d'écartement.

8. Dispositif écarteur selon la revendication 7, **caractérisé en ce que** le prolongement (21) pénètre dans une douille rotative montée sur le boîtier (1) qui, lorsque le prolongement (21) dépasse plus ou moins loin, permet la transmission d'un couple de rotation au prolongement (21) moyennant une complémentarité de formes (32).
